# EUROPEAN PATENT APPLICATION

(11) **EP 3 412 661 A1**
(43) Date of publication of application: **12.12.2018**
(21) Application number: 17382352.7
(22) Date of filing: 08.06.2017
(51) Int. Cl.: C07D 295/096, A61K 31/495, A61P 25/00

(54) **COCRYSTALS OF VORTIOXETINE HYDROBROMIDE AND RESORCINOL**

(71) Applicant: Enantia, S.L., 08028 Barcelona (ES)
(72) Inventor: Jiménez González, Carmen, 08849 SANT CLIMENT DE LLOBREGAT (ES); Tesson, Nicolas, 08902 L'HOSPITALET DE LLOBREGAT (ES)
(74) Representative: ZBM Patents - Zea, Barlocci & Markvardsen

(57) **Abstract**

Cocrystals of vortioxetine hydrobromide and resorcinol, their preparation processes, as well as pharmaceutical compositions comprising them. The cocrystals are useful as active pharmaceutical ingredients of medicaments for the treatment of major depressive disorder.

## Description

### Technical Field

The present invention relates to cocrystals of vortioxetine hydrobromide with resorcinol, processes for their preparation, and their use as medicaments. It also relates to pharmaceutical compositions comprising them.

### Background Art

Vortioxetine is the International Nonproprietary Name (INN) of 1-[2-(2,4-dimethyl-phenylsulfanyl)-phenyl]piperazine and has a CAS number: 508233-74-7. The structure of vortioxetine corresponds to formula (I) below.

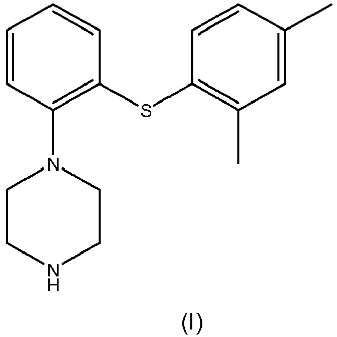

Vortioxetine is a novel antidepressant developed by Takeda Pharmaceuticals and H. Lundbeck A/S jointly, and used for the treatment of major depressive disorder. The drug is marketed under the trade name Brintellix.

WO2003/029232A1 discloses vortioxetine free base and a preparation method thereof, it also mentions its solvates, isomers and pharmaceutically acceptable salts, and their uses in treating affective disorders.

WO2007/144005A1 discloses crystalline vortioxetine base and a variety of crystalline salts thereof, comprising vortioxetine hydrobromide. Specifically, examples 4a to 4i of this document disclose multiple crystalline forms of vortioxetine hydrobromide including α Form, β Form, γ Form, a hemihydrate, and an ethyl acetate solvate. These crystalline forms are difficult to make in a reliable manner since the production processes are especially critical and sensitive because the single crystalline forms are only obtained in pure Form in a quite narrow range of critical parameters using the same solvent. Thus, for instance, the ethyl acetate solvate and the α and β polymorphs are all obtained via crystallizations from ethyl acetate and the γ polymorph and the hemihydrate are both obtained via crystallizations from water.

Besides, solid pharmaceutical compositions must have the ability to maintain the physical, chemical, therapeutic and microbial properties during the time of storage and usage by the patient. According to WO2007/144005A1, Forms α, γ, hemihydrate and ethyl acetate solvate of vortioxetine hydrobromide are not suitable for the preparation of a medicament. For instance, the Form α of vortioxetine hydrobromide is unstable and is transformed to the Form β of vortioxetine hydrobromide at room temperature. The Form γ of vortioxetine hydrobromide is not only very unstable but is also hygroscopic (hygroscopicity under low-humidity conditions: 2.0%). The hemihydrate of vortioxetine hydrobromide is also unstable and hygroscopic. It lost water under high-temperature conditions. Finally, the ethyl acetate solvate of vortioxetine hydrobromide is also unstable. It lost ethyl acetate under high-temperature conditions and transformed to the hydrate under high-humidity conditions (cf. US9562024B2, second column lines 39-42).

Other hydrated crystalline forms of vortioxetine hydrobromide have been disclosed in the art, for instance in WO2014044721A1 (hydrate) and in EP2975032A1 (λ and ω polymorphs which seem to be a sesquihydrate and a monohydrate, respectively). WO2015166379A2 discloses an anhydrous form but it is hygroscopic. There are other documents in the prior art disclosing solvates of vortioxetine or its salts. However, solvates are not suitable forms for the preparation of a medicament due to the strict limits for residual solvents.

Finally, US9562024B2 discloses a vortioxetine hemihydrobromide crystalline form. This crystalline form containing both vortioxetine hydrobromide and vortioxetine base has a much lower kinetic solubility than vortioxetine hydrobromide Form β (example 140). Furthermore, preparation methods described in this patent (examples 1-15) have at least one of these drawbacks: high dilution; preparation by evaporation to dryness step which is not an industrializable process; use of an excess of vortioxetine which is lost during the isolation step.

Different solid forms of a pharmaceutically active ingredient can have different properties and offer certain advantages, for example with regard to solubility or bioavailability. The solubility of an active ingredient is of significance for the choice of solid dosage forms as it may have a direct impact on bioavailability. Salt formation is one of the numerous technologies used for drug solubility enhancement. Furthermore, for solid dosage forms, it is very critical that the drug solubilizes in the gastrointestinal fluid but also that it remains solubilized until it gets absorbed into the bloodstream. If the drug precipitates before getting absorbed, it probably gets excreted unchanged and its effect is diminished.

Vortioxetine has a poor solubility in water, 7.8 mg/L at 25 °C. According to WO2007144005A1, the solubilities of vortioxetine hydrobromide Forms α and β in water are 2 mg/mL and 1.2 mg/mL respectively (see Examples 4b and 4d of WO2007/144005A1, respectively). Salt formation is one known strategy to improve the solubility of a drug. Unfortunately, vortioxetine hydrobromide still presents a poor solubility in water.

Thus, the discovery of new solid forms of an active pharmaceutical ingredient (API) allows improving the characteristics of the pharmaceutical formulations of the active ingredients, since some forms are more suitable for one type of formulation, and other forms for other different formulations. Furthermore, depending on the therapeutic indications, one or another pharmaceutical formulation may be preferred.

Therefore, there is still an ongoing need for new forms of vortioxetine hydrobromide suitable for human administration with enhanced properties, in particular, with enhanced solubility.

### Summary of Invention

Inventors have found that vortioxetine hydrobromide can form cocrystals with resorcinol as a coformer. The cocrystals obtained have a specific stoichiometry.

While active pharmaceutical ingredients (APIs) have been recognized to form crystalline polymorphs, solvates, hydrates and amorphous forms, for a number of years, there is little knowledge about which ones can form cocrystals. By cocrystallizing an API or a salt thereof with a coformer (the second component of the cocrystal), a new solid state form of the API is created having unique properties compared with existing solid forms of the API or its salts. However, cocrystal formation is not predictable, and in fact is not always possible. Moreover, there is no way to predict the properties of a particular cocrystal of a compound until it is formed. In addition, although a number of compounds are prone to form cocrystals, the conditions to obtain such cocrystals tend to be tricky and non-obvious.

Accordingly, an aspect of the present invention relates to the provision of a cocrystal of vortioxetine hydrobromide and resorcinol used as coformer.

In particular, a specific cocrystal of vortioxetine hydrobromide and resorcinol named herein Form I is provided. Advantageously, this cocrystal of vortioxetine hydrobromide and resorcinol is stable (it remained stable after 28 days of exposure under accelerated stability test, cf. example 6) and it shows a higher solubility with respect to the vortioxetine hydrobromide form β (see solubility tests in Example 6) without a spring/parachute effect. The improvement of vortioxetine solubility in the cocrystal of vortioxetine hydrobromide and resorcinol Form I of the present invention is about 50%. The pH is very similar in both cases, remaining constant at 4-5. The maximum solubility is afforded after three hours and then remains constant.

Cocrystals in a biological medium may dissociate. After dissociation, the coformer which is the more water soluble component is drawn out of the crystal lattice into the aqueous medium. Thus, the drug molecules become supersaturated in the aqueous biological medium and this higher energy form of drug as compared to crystalline powder is known as the spring which immediately precipitates to give loosely aggregated clusters. In order to get the benefits from this supersaturated state, it has to be maintained for a sufficient period of time for absorption. The spring effect behaviour of drugs is the dissolution enhancement or boost in percent of dissolved drug due to cocrystal dissociation, and the parachute effect is the decrease in dissolved drug over time due to its inability to remain solubilized.

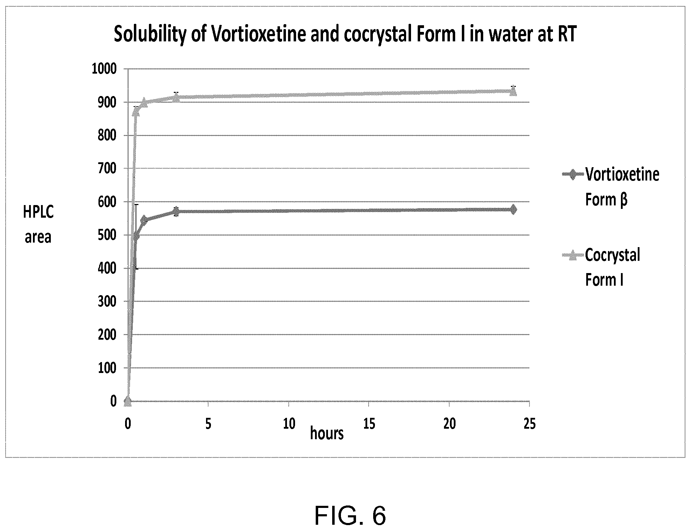

The main advantage of the cocrystal of vortioxetine hydrobromide and resorcinol Form I is the absence of a spring and parachute effect in water (cf. Example 6 and FIG.6) which indicates an unusual solution complex effect of this cocrystal. Thus, on one hand, the cocrystal of vortioxetine hydrobromide and resorcinol Form I is especially advantageous because it exhibits an enhanced solubility without showing an spring and parachute effect (at least during 24 h) and, therefore, it can provide a faster dissolving solid dosage form which remains stable in solution.

Vortioxetine hydrobromide which forms part of the cocrystal of the invention is a well-known drug useful for the treatment of major depressive disorder. Accordingly, another aspect of the present invention relates to the provision of a cocrystal of vortioxetine hydrobromide and resorcinol used as coformer, in particular, a cocrystal of vortioxetine hydrobromide and resorcinol Form I, for use as a medicament.

Another aspect of the present invention relates to the provision of a cocrystal of vortioxetine hydrobromide and resorcinol used as coformer, in particular, a cocrystal of vortioxetine hydrobromide and resorcinol Form I, for use in the prevention and/or treatment of major depressive disorder.

Another aspect of the present invention relates to the provision of a pharmaceutical composition comprising a therapeutically effective amount of a cocrystal of vortioxetine hydrobromide and resorcinol, in particular, of a cocrystal of vortioxetine hydrobromide and resorcinol Form I, together with appropriate amounts of pharmaceutical excipients or carriers.

Finally, another aspect of the present invention, relates to the provision of processes for the preparation of the cocrystals of the invention.

### Brief Description of Drawings

FIG. 1 shows the X-ray powder diffractogram (XRPD) of a cocrystal of vortioxetine hydrobromide and resorcinol Form I.
FIG. 2 shows the proton nuclear magnetic resonance (¹H NMR) of a cocrystal of vortioxetine hydrobromide and resorcinol Form I.
FIG. 3 shows the differential scanning calorimetry (DSC) thermal analysis of a cocrystal of vortioxetine hydrobromide and resorcinol I Form I.
FIG. 4 shows the termogravimetric analysis (TGA) of a cocrystal of vortioxetine hydrobromide and resorcinol Form I.
FIG. 5 shows the XRPD of vortioxetine hydrobromide form β of reference example 1 in comparison to the XRPD diffractogram of Vortioxetine•HBr Form β described in the patent WO2007144005A1.
FIG. 6 shows the solubility curve of the cocrystal of vortioxetine hydrobromide and resorcinol Form I.

### Detailed description of the invention

### Definitions:

The term "cocrystal" refers herein to a crystalline entity with at least two different components constituting the unit cell at room temperature (20-25 °C) and interacting by weak interactions. Thus, in a cocrystal the active pharmaceutical ingredient crystallizes with one or more neutral components. The cocrystal may include one or more solvent molecules in the crystal lattice.

The term "weak interaction" refers herein as an interaction which is neither ionic nor covalent, and includes for example: hydrogen bonds, van der Waals interactions, and π-π stacking.

The term "solvate" is to be understood as meaning any form of the cocrystal in which the compound has attached to it via non-covalent binding solvent molecules. When the solvent is water the solvate is a hydrate.

When a ratio of components of the cocrystals of the invention is specified it refers to the molar ratio between vortioxetine hydrobromide and resorcinol. A ratio of the cocrystal of vortioxetine hydrobromide and resorcinol 2:1 means a molar ratio of vortioxetine hydrobromide:resorcinol.

When values of peaks of an X-ray diffractogram are given it is said that they are "approximate" values. It should be understood that the values are the ones shown in the corresponding lists or tables ± 0.1 degrees 2 theta measured in an X-ray diffractometer with Cu-K_{α} radiation λ=1.5406 Å.

The term "room temperature" as disclosed herein refers to a temperature of the environment, without heating or cooling, and is generally comprised of from 20 to 25 °C.

For the purposes of the invention, any ranges given include both the lower and the upper end-points of the range. Ranges given, such as temperatures, times, and the like, should be considered approximate, unless specifically stated.

The expression "cocrystal obtainable by" is used here to define each specific cocrystal of the invention by the process for obtaining it and refers to the product obtainable by any of the corresponding processes disclosed herein. For the purposes of the invention the expressions "obtainable", "obtained" and equivalent expressions are used interchangeably, and in any case, the expression "obtainable" encompasses the expression "obtained".

The terms "wet grinding" and "liquid assisted grinding" are equivalent and refer to a technique which consists of milling or grinding the product or mixture with some drops of solvent added. Neat and liquid-assisted grinding are techniques that can be employed in order to produce cocrystals. In neat (dry) grinding, cocrystal formers are ground together manually using a mortar and pestle, using a ball mill, or using a vibratory mill. In liquid-assisted grinding, or kneading, a small or substoichiometric amount of liquid (solvent) is added to the grinding mixture.

The term "therapeutically effective amount" as used herein, refers to the amount of a compound that, when administered, is sufficient to prevent development of, or alleviate to some extent, one or more of the symptoms of the illness to be treated. The particular dose of compound administered according to this invention will of course be determined by the particular circumstances surrounding the case, including the compound administered, the route of administration, the particular condition being treated, and the similar considerations.

The term "pharmaceutical composition" refers to a mixture of a compound disclosed herein with other chemical components, such as diluents or carriers. The pharmaceutical composition facilitates administration of the compound to an organism.

The term "pharmaceutically acceptable excipients or carriers" refers to pharmaceutically acceptable material, composition or vehicle, such as liquid or solid filler, diluent, excipient, solvent, or encapsulating material. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "treatment" is meant to include alleviating or eradicating a disorder, disease, or condition, or one or more of the symptoms associated with the disorder, disease or condition, or alleviating or eradicating the cause(s) of the disorder, disease, or condition itself.

As mentioned above, provided is a cocrystal of vortioxetine hydrobromide and resorcinol. Resorcinol is a pharmaceutically acceptable coformer which is not an active pharmaceutical ingredient. It has the following formula and is a commercial product.

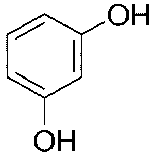

A preferred cocrystal is that identified herein as a cocrystal of vortioxetine hydrobromide and resorcinol named Form I. This cocrystal has been characterized by XRPD, ¹H NMR DSC, and TGA as described below.

The XRPD analysis disclosed herein was performed at ambient conditions on a PANalytical X'Pert PRO θ-θ diffractometer of 240 mm of radius in reflection geometry, equipped with Cu Kα radiation and a PIXcel detector, operated at 45 kV and 40 mA. The sample was mounted on a zero background silicon sample holder and allowed to spin at 0.25 rev/s during the data collection. The measurement angular range was 3.0-40.0° (2θ) with a step size of 0.013°. The scanning speed was 0.328°/s (10.2 s/step).

The cocrystal of vortioxetine hydrobromide and resorcinol Form I may be characterized by having an X-ray diffractogram that comprises peaks at approximately 9.0 and 17.9 degrees 2 theta (Cu-K_{α} radiation, A = 1.5406 Å). Specifically, this cocrystal may be characterized by additionally exhibiting in the powder X-ray diffractogram, peaks at 12.1, 19.1 and 23.6 degrees 2 theta (Cu-K_{α} radiation, λ = 1.5406 Å). More specifically, this cocrystal may be characterized by additionally exhibiting in the powder X-ray diffractogram, peaks at 14.0, 14.5, 18.1, 18.3, 19.3, 21.4, 21.9, 24.2, 24.4, 24.7, 25.9, 26.4, and 27.0 degrees 2 theta (Cu-K_{α} radiation, λ = 1.5406 Å). Even more specifically, this new cocrystal Form I may be characterized by exhibiting in the powder X-ray diffractogram a pattern of peaks, expressed in 2 theta units in degrees, 2θ (°), which is shown in Table 1.

**Table 1: List of selected peaks of XRPD (only peaks with relative intensity greater than or equal to 1% are indicated):**

| Pos. [°2θ] | d-spacing [Å] | Rel. Int. [%] |
|---|---|---|
| 6,0 | 14,8 | 6 |
| 84 | 10,5 | 7 |
| 9,0 | 9,8 | 40 |
| 12,1 | 7,3 | 10 |
| 14,0 | 6,3 | 31 |
| 14,5 | 6,1 | 19 |
| 16,4 | 5,4 | 11 |
| 17,2 | 5,1 | 16 |
| 17,9 | 4,9 | 70 |
| 18,1 | 4,9 | 64 |
| 18,3 | 4,8 | 17 |
| 19,1 | 4,6 | 94 |
| 19,3 | 4,6 | 58 |
| 21,4 | 4,2 | 36 |
| 21,7 | 4,1 | 15 |
| 21,9 | 4,1 | 19 |
| 23,6 | 3,8 | 100 |
| 23,8 | 3,7 | 11 |
| 24,2 | 3,7 | 17 |
| 24,4 | 3,6 | 28 |
| 24,7 | 3,6 | 38 |
| 25,0 | 3,6 | 10 |
| 25,3 | 3,5 | 9 |
| 25,9 | 3,4 | 20 |
| 26,4 | 3,4 | 34 |
| 27,0 | 3,3 | 28 |
| 27,6 | 3,2 | 10 |
| 28,5 | 3,1 | 12 |
| 29,2 | 3,1 | 14 |
| 29,4 | 3,0 | 15 |
| 30,2 | 3,0 | 3 |
| 30,9 | 2,9 | 5 |
| 31,8 | 2,8 | 12 |
| 33,0 | 2,7 | 12 |
| 33,5 | 2,7 | 11 |
| 34,1 | 2,6 | 6 |
| 35,9 | 2,5 | 6 |
| 36,7 | 2,4 | 4 |
| 39,4 | 2,3 | 3 |

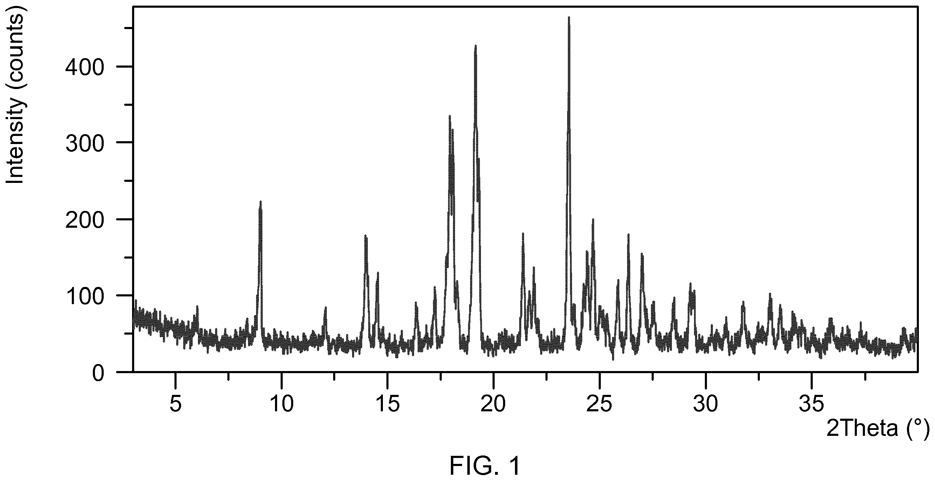

This cocrystal Form I of the present invention may be further characterized by an X-ray diffractogram as in FIG. 1.

The ¹H NMR disclosed herein was recorded in deuterated chloroform In a Varian Mercury 400 spectrometer, equipped with a broadband probe ATB 1 H/19F/X of 5 mm. Spectra were acquired dissolving 5-10 mg of sample in 0.6 mL of MeOD-d4 solvent.

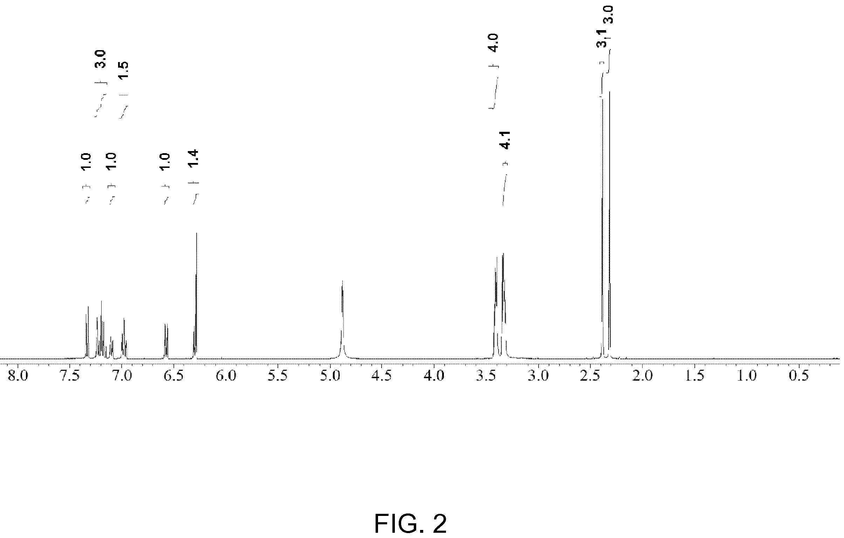

The cocrystal of vortioxetine hydrobromide and resorcinol Form I of the present invention may be characterized by the following ¹H NMR spectra, 400 MHz. 7.33 (d, 1 H, *J* = 7.8 Hz, ArH); 7.26-7.14 (m, 3H, ArH); 7.10 dd, *J* = 1.6 Hz, *J* = 7.8 Hz, 1 H, ArH); 7.01-6.94 (m, 1.5H, ArH); 6.57 (dd, *J* = 1.2 Hz, *J* = 7.8 Hz, 1 H); 6.32-6.26 (m, 1.5H, ArH); 3.45-3.28 (m, 8H, CH₂); 2.38 (s, 3H, CH₃); 2.31 (s, 3H, CH₃). This cocrystal Form I may be further characterized by a ¹H NMR spectra as in FIG. 2.

The resonance signals of the ¹H NMR spectra are coherent with vortioxetine hydrobromide and resorcinol in a 2:1 ratio taking into account the NMR integration error; and without presence of any solvent.

Thus, the cocrystal Form I has a molar ratio of vortioxetine hydrobromide:resorcinol determined by ¹H NMR integration of about 2:1. The term "about" means that small variations can be obtained due to the level of sensibility of ¹H NMR.

DSC analysis disclosed herein was performed with a Mettler Toledo DSC2. 4.3700 mg of Form I was weighed into a 40 µL aluminium crucible with a pinhole lid and heated from 25 to 300 °C at a rate of 10 °C/min, under nitrogen (50 mL/min).

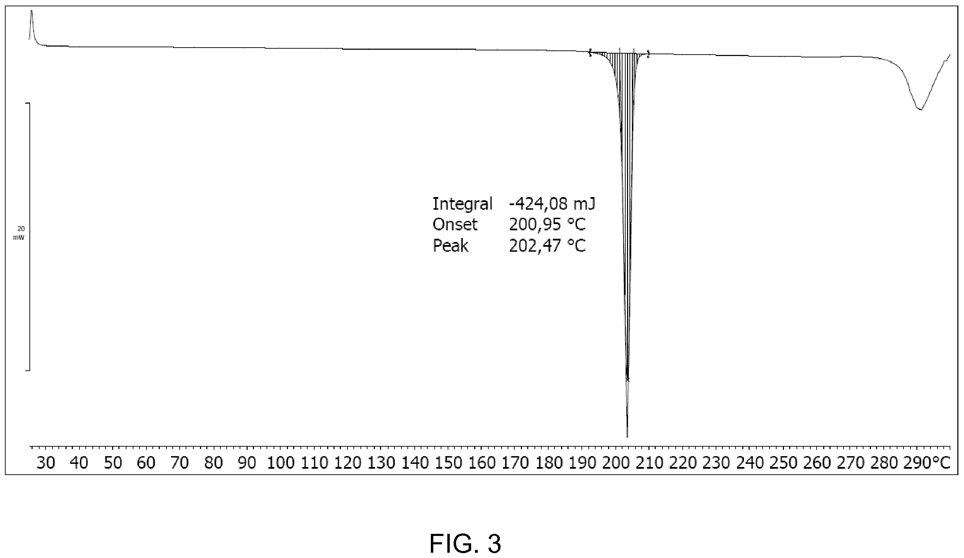

The cocrystal of vortioxetine hydrobromide and resorcinol Form I of the present invention may be characterized by a DSC analysis that shows an endothermic event with an onset at about 201 °C which corresponds to the melting point of the cocrystal of vortioxetine hydrobromide and resorcinol Form I (cf. FIG. 3).

TG analysis disclosed herein was recorded in a thermogravimetric analyzer Mettler Toledo TGA/SDTA851e. A sample of 4.3110 mg was weighed into a 100 µL alumina crucible and sealed with a lid. The lid was automatically punched by the robot just before the analysis. Samples were heated at 10 °C/min from 25 to 300 °C, under nitrogen (50 mL/min).

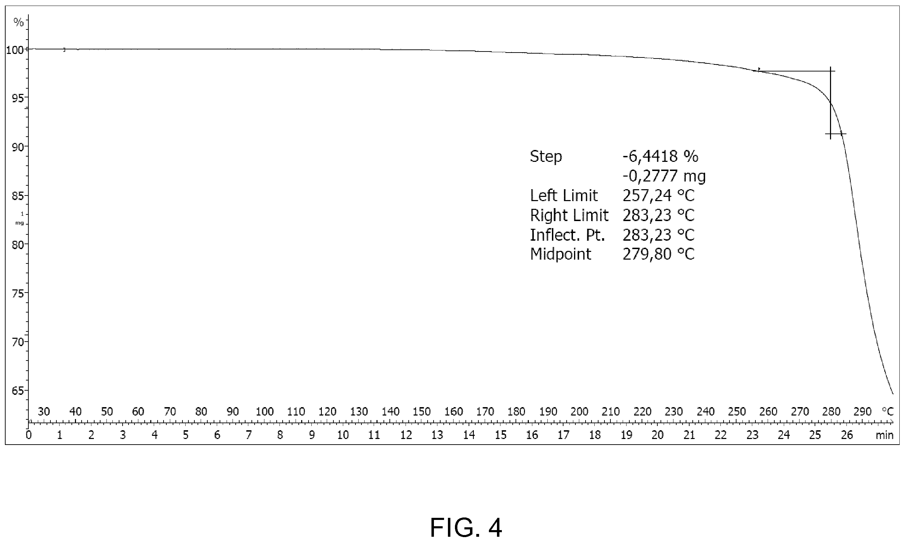

TGA analysis of the cocrystal of vortioxetine hydrobromide and resorcinol Form I shows no weight loss before the melting point (cf. FIG. 4).

The cocrystal of vortioxetine hydrobromide and resorcinol Form I can be readily prepared and is easy to scale-up.

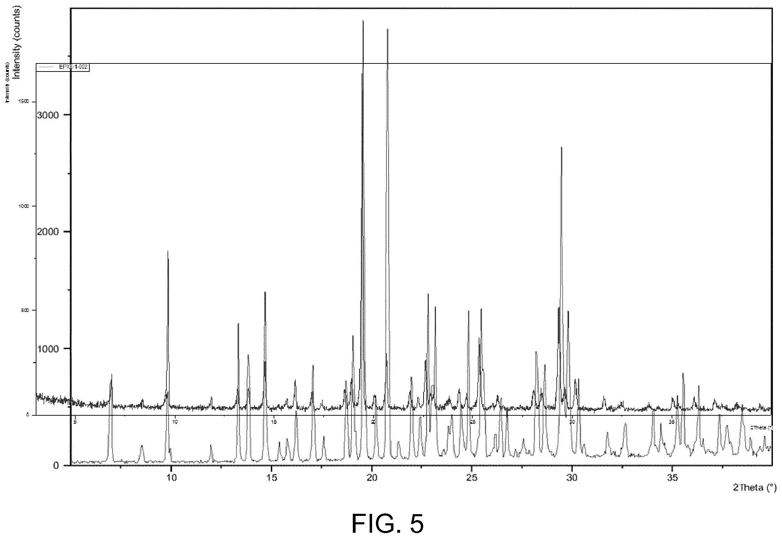

Vortioxetine hydrobromide form β can be used as starting material of the processes disclosed herein to obtain the cocrystal of the invention. Form β was identified by XRPD (see FIG. 5).

The cocrystal of vortioxetine hydrobromide and resorcinol Form I can be obtained with a degree of purity equal to or greater than 99% a/a by HPLC, preferably with a degree of purity equal to or greater than 99.5% a/a by HPLC, more preferably, equal to or greater than 99.9% a/a by HPLC.

HPLC conditions to determine the vortioxetine hydrobromide purity such as those described in the article J Pharm Biomed Anal. 2016, 117, 325-32 can be used to determine the degree of purity. Chromatographic studies were performed on a Waters 2695 system (Milford, MA, USA) equipped with a 2996 photo diode array detector linked to an Empower data-handling system. The separation was performed on a Shimadzu Inertsil ODS-3 C18 column (250 mm × 4.6 mm, 5 µm) (Tokyo, Japan). Mobile phase A was 0.05% trifluoroacetic acid in water, and phase B was ACN. The HPLC gradient program was as follows: Time (min): A/B (v/v); T0 80/20, T30 60/40, T50 20/80, T60 20/80, T65 80/20, T70 80/20. The UV detector wavelength was set at 230 nm. The flow rate was 1.0 mL/min, and the column temperature was maintained at 35 °C. The samples were prepared in a water and acetonitrile mixture (80:20, v/v) at 0.5 mg/mL concentration, and 20 µL of sample solution was injected into the HPLC system for analysis.

The cocrystal of vortioxetine hydrobromide and resorcinol Form I as defined above may be prepared by a process which comprises the following steps: (a) wet grinding of a mixture of vortioxetine hydrobromide Form β and resorcinol in water at room temperature; and (b) isolating the compound thus obtained.

It can also be obtained by wet-grinding of a mixture of vortioxetine hydrobromide Form β and resorcinol in acetonitrile, methanol, ethyl acetate, acetone, tetrahydrofuran, chloroform, toluene and heptane.

Alternatively, the cocrystal of vortioxetine hydrobromide and resorcinol Form I may also be prepared by a process which comprises the following steps: (a) slurrying a mixture of vortioxetine hydrobromide Form β and resorcinol in water at room temperature, optionally, seeding the mixture with the cocrystal of vortioxetine hydrobromide and resorcinol Form I, and (b) isolating the compound thus obtained. The cocrystal formed by this method may be separated from the medium at room temperature or at a lower temperature, for instance, the mixture may be cooled to a temperature around 0-5 °C before separating the product. The cocrystal formed may be separated by filtration or other suitable techniques as known to a skilled person in the art. The compound isolated in any of the previous processes can be dried at room temperature, preferably under vacuum. Generally, the vacuum is comprised of 0.5 to 3 mbar.

The same process can be carried out in chloroform as solvent instead of water.

Alternatively, the cocrystal of vortioxetine and resorcinol Form I may also be prepared by a process which comprises its crystallization from a solution of vortioxetine hydrobromide and resorcinol in a solvent selected from the group consisting of water, isopropanol, and a mixture thereof; and subsequently isolating the compound thus obtained.

In a particular embodiment of this previous process, the cocrystal of vortioxetine and resorcinol Form I can be prepared by a process which comprises the following steps: (a) vortioxetine·HBr solution preparation in a hot selected solvent by dissolution of vortioxetine·HBr or by generating in solution vortioxetine·HBr from vortioxetine and HBr; (b) adding a solution of resorcinol; and (c) cooling the solution thus obtained to room temperature, and optionally to 0-5 °C; and (d) isolating the compound thus obtained. The solvent can be selected from the group consisting of water, isopropanol, and a mixture of thereof. Preferably, the solvent used is water.

If desired, the solution of the previous process in step (c) can be seeded with the cocrystal of vortioxetine hydrobromide and resorcinol Form I.

The cocrystal of vortioxetine hydrobromide and resorcinol of the present invention may also be defined by its preparation process. Accordingly, this aspect of the invention can be formulated as vortioxetine hydrobromide and resorcinol cocrystal as defined above, obtainable by any of the previous processes, optionally including any preferred or particular embodiment of the process and possible combinations of some of the process features disclosed above.

Likewise, a cocrystal of vortioxetine hydrobromide and resorcinol Form I obtainable by any of the processes disclosed above for its obtention is also part of the invention.

Pharmaceutical compositions comprising a therapeutically effective amount of a cocrystal of vortioxetine hydrobromide and resorcinol, in particular, of the cocrystal of vortioxetine hydrobromide and resorcinol Form I, together with appropriate amounts of pharmaceutical excipients or carriers are also part of the invention. They can be prepared according to methods well known in the state of the art. The appropriate excipients and/or carriers, and their amounts, can readily be determined by those skilled in the art according to the type of formulation being prepared.

It is also part of the invention the provision of a cocrystal of vortioxetine hydrobromide and resorcinol, in particular, the cocrystal of vortioxetine hydrobromide and resorcinol Form I, for use in the prevention and/or treatment of major depressive disorder. This aspect can also be formulated as the use of a cocrystal of vortioxetine hydrobromide and resorcinol, in particular, the cocrystal of vortioxetine hydrobromide and resorcinol Form I, for the preparation of a medicament for the prophylactic and/or therapeutic treatment of major depressive disorder in a mammal, including a human. The invention also relates to a method of treatment and/or prophylaxis of major depressive disorder, said method comprises the administration to a human, suffering from or being susceptible to suffer from major depressive disorder, a therapeutically effective amount of a cocrystal of vortioxetine hydrobromide and resorcinol, in particular, a cocrystal of vortioxetine hydrobromide and resorcinol Form I, together with pharmaceutically acceptable excipients or carriers.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Reference Example 1: Preparation of vortioxetine•HBr Form β

To a round-bottomed flask equipped with magnetic stirring containing vortioxetine (8.00 g, 26.81 mmol) in EtOAc (120 mL) was added HBr (48%-wt) (3.05 mL, 8.78 mmol, 1 eq.). The resulting solution was stirred for one hour at room temperature. The precipitated solid was filtered through a sinter funnel (porosity n°3), washed with 2 x 5 mL of EtOAc and dried under vacuum at room temperature giving vortioxetine·HBr Form β as a white solid (9.80 g, 96%). The XRPD diffractogram of the recovered solid matches with the XRPD diffractogram of vortioxetine·HBr Form β described in the patent WO2007144005A1. ¹H NMR spectrum displayed resonance signals coherent with vortioxetine structure. Moreover, significant shifts in the resonance signals compared to the ¹H NMR spectrum of vortioxetine base were observed indicating that the hydrobromide salt was formed.

### Example 2: Preparation of a cocrystal of vortioxetine•HBr and resorcinol Form I (2:1) by grinding

To a 2 mL Eppendorf tube containing vortioxetine·HBr Form β (20 mg, 0.053 mmol), resorcinol (6 mg, 0.053, 1 eq.) and 1-2 drops of water were added three stainless steel grinding balls before milling for 45 minutes at a rate of 30 Hz (3 cycles of 15 minutes) with a Retsch Ball Mill MM 400. Drying under vacuum (approx. 1-2 mbar) at room temperature gave the cocrystal of vortioxetine hydrobromide and resorcinol Form I contaminated with traces of resorcinol as a white solid.

Slurring a mixture of vortioxetine·HBr Form β and resorcinol (stoichiometry 1:2, i.e. an excess of resorcinol is used) in water afforded pure Form I.

The same results were obtained with acetonitrile, methanol, ethyl acetate, acetone, tetrahydrofuran, and chloroform as solvent.

### Example 3: Preparation of a cocrystal of vortioxetine•HBr and resorcinol Form I (2:1) by slurrying

To a round-bottomed flask equipped with magnetic stirring containing a mixture of vortioxetine·HBr Form β (3.00 g, 7.908 mmol) and resorcinol (1.74 g, 15.820 mmol, 2 eq.), was added water (45 mL). The resulting mixture was seeded with the cocrystal of vortioxetine·HBr and resorcinol Form I and stirred at room temperature for 15 hours. The crude was filtered through a sinter funnel (porosity n°3), washed with 2 x 3.0 mL of water and dried under vacuum at room temperature until constant weight giving the cocrystal of vortioxetine·HBr and resorcinol Form I as a white solid (3.17 g, 92%). XRPD analysis showed the formation of the new crystalline Form I (Cf. FIG.1). The ¹H NMR spectrum displayed resonance signals coherent with vortioxetine structure and 0.5 equivalents of resorcinol, without the presence of any solvent (cf. FIG. 2). DSC analysis shows an endothermic event with an onset at about 201 °C which should correspond to the melting point of Form I (cf. FIG.3). The TG analysis of Form I shows no weight loss before the melting point (cf. FIG. 4).

The same process can be carried out without seeding, yielding to the cocrystal of vortioxetine·HBr and resorcinol Form I.

### Example 4: Stability teats

The stability of the cocrystal of vortioxetine hydrobromide and resorcinol Form I and vortioxetine·HBr Form β used as starting material, were evaluated by atmospherization of a sample exposed on the XRPD sample holder under accelerated stability conditions according to ICH guidelines (75±5% RH, 40 °C). The samples were analyzed by XRPD to observe if the crystalline phase was stable (see Table 2).

**Table 2:**

| Days (40 °C / 75±5% RH) | XRPD | |
|---|---|---|
| | Cocrystal Form I | Vortioxetine·HBr Form β |
| 7 | stable | stable |
| 14 | stable | stable |
| 21 | stable | stable |
| 28 | stable | stable |

The Vortioxetine•HBr Form β and the cocrystal of vortioxetine·HBr and resorcinol Form I remained stable after 28 days of exposure under accelerated stability conditions.

### Example 5: Stability competition tests

An equal quantity (250 mg) of vortioxetine hemihydrobromide crystalline form prepared following example 3 of US9562024B2, and either Vortioxetine•HBr Form β or Form I of the present invention were added to 3 mL water to produce a suspension. Both suspensions were slurried for 5 days at room temperature, and the XRPD characterization analyses were performed.

**Table 3:**

| Days (RT - water slurrying) | XRPD | |
|---|---|---|
| | Form I + hemihydrobromide | Form β + hemihydrobromide |
| 1 | No change | No change |
| 5 | No change | Hemihydrobromide + Form β traces |

| | | |
|---|---|---|
| XRPD showed the transformation of Form β into vortioxetine hemihydrobromide crystalline form as described in US9562024B2, while Form I remained stable in the presence of vortioxetine hemihydrobromide crystalline form. | | |

### Example 6: Solubility tests

A comparative kinetic solubility study of the cocrystal of vortioxetine·HBr and resorcinol 2:1 Form I and with vortioxetine·HBr Form β was performed in water at room temperature (RT). The relative solubility of vortioxetine was obtained by comparison of the vortioxetine peak area in the HPLC analyses of the mother liquors.

The solubility tests were performed in a minireactor HME-R, provided with a 250 mL vessel, a temperature sensor and mechanical stirring. Cocrystal of vortioxetine hydrobromide and resorcinol Form I of the present invention and vortioxetine·HBr Form β were previously ground in a mortar in order to minimize the particle size effect (no crystalline transformation took place as confirmed by XRPD).

1 g of each material was stirred in water (150 ml, 15 V) at 350 rpm. Aliquots of ca. 5 ml of suspension were filtered in a sintered glass funnel (#4) at different times: 30, 60, 180 min and 24 h. After filtration, the pH of the mother liquor was determined. 1 mL of mother liquor was measured with a pipette and diluted to 10 mL with water in a volumetric flask. A portion of this solution was filtered again through a 0.20 µm nylon filter and directly analyzed by HPLC (2 injections for each sample). The filtered solids were analyzed by XRPD, without a drying step, to determine the crystalline phase corresponding to the measured solubility.

The HPLC conditions were the following:

| | |
|---|---|
| Column: | Zorbax Eclipse XDB-C18, 5 µm, 4.6x150 mm |
| Mobile phase: | 0.05% aqueous TFA : ACN (60:40) |
| Temperature: | RT |
| Flow rate: | 1 mL/min |
| Wavelength: | 230 nm |
| Injection: | 1 µL |
| Run time: | 15 min |

The kinetic solubility of cocrystal vortioxetine hydrobromide and resorcinol Form I and vortioxetine·HBr Form β at room temperature in water was determined in duplicate. The results of the solubility studies at room temperature in water are shown in the following tables (see Table 4 for cocrystal Form I and Table 5 for vortioxetine·HBr Form β).

**Table 4: Results of cocrystal Form I solubility study at room temperature in water**

| Time | Analysis 1 | | | Analysis 2 | | | Average Vortioxetine area | SD |
|---|---|---|---|---|---|---|---|---|
| | Average Form I (Vortioxetine area) | pH | XRPD | Average Form I (Vortioxetine area) | pH | XRPD | | |
| 30 min | 863 | 5.0 | Form I | 883 | 4.9 | Form I | 873 | 14 |
| 60 min | 899 | 4.9 | Form I | 899 | 4.8 | Form I | 899 | 0 |
| 180 min | 926 | 3.8 | Form I | 905 | 4.7 | Form I | 915 | 15 |
| 24 h | 927 | 4.7 | Form I | 943 | 4.1 | Form I | 935 | 11 |

**Table 5: Results of Vortioxetine•HBr Form β solubility study at room temperature in water**

| Time | Analysis 1 | | | Analysis 2 | | | Average Vortioxetine area | SD |
|---|---|---|---|---|---|---|---|---|
| | Average Form β (Vortioxetine area) | pH | XRPD | Average Form β (Vortioxetine area) | pH | XRPD | | |
| 30 min | 564 | 4.9 | Form β | 427 | 5.3 | Form β | 495 | 97 |
| 60 min | 550 | 4.8 | Form β | 537 | 5.1 | Form β | 544 | 9 |
| 180 min | 579 | 4.8 | Form β | 561 | 5.1 | Form β | 570 | 12 |
| 24 h | 572 | 5.6 | Form β | 581 | 5.5 | Form β | 576 | 7 |

The average of the HPLC area values of vortioxetine and the cocrystal of the invention, named Form I were plotted against time (see FIG.6).

A spring and parachute effect is not seen in any case due to the stability of both cocrystal Form I and vortioxetine·HBr Form β in water. Both crystalline forms remain unchanged after 24 hours in water dispersion. The maximum of solubility is afforded in both cases after three hours. The improvement of vortioxetine solubility in cocrystal Form I is about 50%. The pH is very similar in both cases, remaining constant at 4-5.

### Citation List

### Patent Literature

- WO2003/029232A1
- WO2007/144005A1
- WO2014044721A1
- US2016015706A1
- WO2015166379A2
- WO2015035802A1

## Claims

1. A cocrystal of vortioxetine hydrobromide and resorcinol.

2. The cocrystal according to claim 1, which is **characterized by** having an X-ray diffractogram that comprises peaks at 9.0 and 17.9 degrees 2 theta at a Cu-K_{α} radiation, λ = 1.5406 Å.

3. The cocrystal according to claim 2, which is **characterized by** having an X-ray diffractogram that comprises peaks at approximately 12.1, 19.1 and 23.6 degrees 2 theta at a Cu-K_{α} radiation, A = 1.5406 Å.

4. The cocrystal according to claim 3, which is **characterized by** having an X-ray diffractogram that comprises peaks at approximately 14.0, 14.5, 18.1, 18.3, 19.3, 21.4, 21.9, 24.2, 24.4, 24.7, 25.9, 26.4, and 27.0 degrees 2 theta at a Cu-K_{α} radiation, λ = 1.5406 Å.

5. The cocrystal according to any of the claims 2-4, which is **characterized by** having a DSC analysis which shows an endothermic event with an onset at about 201 °C.

6. The cocrystal according to any of the claims 1-5, wherein the molar ratio vortioxetine hydrobromide: resorcinol is about 2:1.

7. A cocrystal of vortioxetine hydrobromide and resorcinol as defined in any of the claims 1-6, for use as a medicament.

8. A cocrystal of vortioxetine hydrobromide and resorcinol as defined in any of the claims 1-6, for use in the prevention and/or treatment of major depressive disorder.

9. A pharmaceutical composition comprising a cocrystal of vortioxetine hydrobromide and resorcinol as defined in any of the claims 1-6, together with appropriate amounts of pharmaceutical excipients or carriers.

10. A process for the preparation of a cocrystal of vortioxetine hydrobromide and resorcinol cocrystal as defined in any of the claims 1-6, which comprises slurrying a mixture of vortioxetine hydrobromide form β and resorcinol in water at room temperature for the necessary period of time to form the cocrystal; and subsequently isolating the compound thus obtained.

11. The process according to claim 10, wherein the isolation of the cocrystal comprises filtering the product thus obtained and drying it at room temperature under vacuum.

12. The process according to any of the claims 10-11, wherein the cocrystal of vortioxetine hydrobromide and resorcinol is the Form I as defined in any of the claims 3-6, and the process comprises using 2 moles of resorcinol per mol of vortioxetine hydrobromide.

13. A process for the preparation of a cocrystal of vortioxetine hydrobromide and resorcinol Form I, as defined in any of the claims 1-6, which comprises its crystallization from a solution of vortioxetine hydrobromide and resorcinol in a solvent selected from the group consisting of water, isopropanol, and a mixture thereof; and subsequently isolating the compound thus obtained.
